# NEUE EUROPÄISCHE PATENTSCHRIFT

(11) **EP 1 379 470 B2**
(45) Veröffentlichungstag und Bekanntmachung des Hinweises auf die Entscheidung über den Einspruch: **14.03.2012**
(45) Hinweis auf die Patenterteilung: 27.06.2007
(21) Anmeldenummer: 02732550.5
(22) Anmeldetag: 03.04.2002
(51) Int. Cl.: C01G 49/02, C09C 1/24

(54) **EISENOXIDE MIT HÖHEREM VEREDELUNGSGRAD**
IRON OXIDES WITH A HIGHER DEGREE OF REFINING
OXYDES DE FER PRESENTANT UN DEGRE ELEVE D'AFFINAGE

(30) Priorität: 10.04.2001 DE 10117996; 09.11.2001 DE 10154718
(43) Veröffentlichungstag der Anmeldung: 14.01.2004
(73) Patentinhaber: BASF SE, 67056 Ludwigshafen (DE)
(72) Erfinder: WALSDORFF, Christian, 67059 Ludwigshafen (DE); BAIER, Michael, 68161 Mannheim (DE); KÖRNER, Reinhard, 67227 Frankenthal (DE); HARTH, Klaus, 67317 Altleiningen (DE); VORBERG, Gerald, 67346 Speyer (DE); RUPPEL, Wilhelm, 68163 Mannheim (DE)
(86) Internationale Anmeldenummer: PCT/EP2002/003672
(87) Internationale Veröffentlichungsnummer: WO 2002/083569

(56) Entgegenhaltungen:
- EP-A- 0 419 964
- WO-A-95/25069
- US-A- 4 436 681
- US-A- 5 597 547
- US-A- 5 911 967
- DATABASE WPI Section Ch, Week 198937 Derwent Publications Ltd., London, GB; Class E31, AN 1989-266659 XP002216178 & JP 01 192731 A (SUMITOMO METAL IND LTD), 2. August 1989 (1989-08-02)
- DATABASE WPI Section Ch, Week 199011 Derwent Publications Ltd., London, GB; Class E31, AN 1990-080254 XP002216007 & JP 02 034519 A (MIZUWATARI H), 5. Februar 1990 (1990-02-05)
- PATENT ABSTRACTS OF JAPAN vol. 010, no. 345 (C-386), 20. November 1986 (1986-11-20) & JP 61 146719 A (NISSHIN STEEL CO LTD), 4. Juli 1986 (1986-07-04)

## Beschreibung

Die vorliegende Erfindung betrifft ein Verfahren zur Veredelung von Eisenoxid, wobei daß Eisenoxid bei mindestens 700°C kontinuierlich calciniert wird.

Aus der EP-A-1 027 928 ist ein Katalysator, insbesondere zur Dehydrierung von Ethylbenzol zu Styrol beschrieben, der unter Vierwendung eines durch Sprühr8stung einer Eisensalzlösunar, insbesondere salzsaurer Eisenlösungen (Ruthner-Verfahren), erhaltenen Eisenoxids hergestellt wird. Nachteil solcher Eisenoxide ist der darin enthaltene hohe Restgehalt an Chlorid.

Aus der EP-A-797 481 sind Eisenoxide als Ausgangsmaterial für Katalysatoren, insbesondere zur Dehydrierung von Ethylbenzol zu Styrol, bekannt, die durch Mischen mit einer weiteren Metallverbindung und anschließendem Calcinieren restrukturiert werden, und die sehr kleine BET-Oberflächen haben. Nachteil einer solchen Reatrukturierung ist die Kontamination des Eisenoxids durch die zugesetzten Metallverbindung.

Aus der JP-A-61-72601 ist ein Wirbelschichtverfahren zur Spaltung schwerer Kohlenwasserstoffe in leichtere (katalytisches Cracken) unter Verwendung eines Katalysators bekannt, wobei der pulverförmige Katalysator durch Aufschlämmen eines Eisenoxidpulvers mit Wasser, Sprühtrocknung und schließlich Calcinierung bei Temperaturen zwischen 1200 und 1600°C hergestellt wird. Nachteilig an diesem Verfahren ist der erhebliche Aufwand bei der Herstellung und die hohen calcinierungatemperaturen.

Aus der EP-A-827 488 ist eine Reduzierung des Restchloridgehalts in Eisenoxiden, inbesondere in Eisenoxiden aus der Aufarbeitung salzsaurer Beizlösungen, durch Mischen des Eisenoxids mit einer hydratisierten Metallverbindung und anschließende Calcinierung beschrieben. Nachteil dieses Verfahrens ist der relativ große Aufwand bei der Herstellung.

Aus der US-A-4, 134, 858 ist bekannt zur Herstellung von Styrol-Katalysatoren eingesetztes Eisenoxid vorab bei 800°C zu rösten. Ein im Eisenoxid vorhandener Restchloridgehalt lässt sich mit dieser Methode allerdings nur unzureichend verringern.

Aus der US-A-2,414,585 ist bekannt, Eisenoxid für die Herstellung von Dehydrierkatalysatoren vorzuaalcinieren. Dabei soll ein Eisenoxid mit einer BET-Oberfläche von < 8 m³/g und bevorzugt von ungefähr 4 m²/g erhalten werden. Solche Katalysatoren lassen zu wünschen übrig.

WO95/25069 A offenbart ein Verfahren zur Veredelung von Eisenoxiden, bei dem das Eisenoxid zunächst mit Schwefelsäure umgesetzt, filtriert, getrocknet und anschließend bei 825 °C calciniert wird, wobei das erhaltene Eisenoxid 390 ppm Chlorid enthält.
In Beispiel C-1 wird ein Eisenoxid ohne Zugabe von Additiven bei 825°C calciniert, wobei der Restchloridgehalt bei 810 ppm liegt. WO95/25069 A rät demnach von einer Calcinierung ohne zusätzlichen Verfahrensschritt und ohne den Zusatz weiterer Stoffe ab.

JP 61-146719 beschreibt ein Verfahren zur Entfernung von Chloridionen aus Eisenoxid bei 300 bis 600 °C unter reduziertem Druck. Ein kontinuierliches Verfahren wird nicht offenbart.

US 4,436,681 offenbart ein Verfahren zur Herstellung von Eisenoxid durch Sprühtrocknung von Eisenchloridlösungen und anschließende Calcinierung bei 900 bis 1300 °C. Vor der Calcinierung muss das Material kompaktiert werden, wodurch das Verfahren erschwert wird.

US 5,597,547 beschreibt ein Verfahren zur Verringerung des Chloridgehalts in Eisenoxiden, bei dem das Eisenoxid zunächst mit kristallwasserhaltigem gelben Eisenoxid und Wasser zu vermischen und anschließend zu trocknen und zu calcinieren ist. Diese zusätzlichen Verfahrensschritte machen das Verfahren relativ aufwendig,

US b,991,967 offenbart ein Verfahren und eine Vorrichtung zur Herstellung von Eisenoxid aus Eisenchloridlösungen, umfassend die Sprühröstung der Lösung, die Abkühlung der erhaltenen Granalien auf unter 450 °C, den Transfer der Granalien in einen anderen Apparat und die anschließende Behandlung mit Dampf bei Temperaturen im Bereich von 325 bis 380 °C. Auch dieses Verfahren ist recht aufwendig und nicht kontinuierlich durchführbar.

Der vorliegende Erfindung lag daher die Rufgabe zugrunde, den zuvor genannten Nachteilen abzuhelfen.

Demgemäß wurde ein neues und verbessertes Verfahren zur Veredelung von Eisenoxid gefunden, welches dadurch gekennzeichnet ist, dass man Eisenoxide aus der Sprühröstung von salzsauren Beizlösungen einsetzt und diese ohne mechanische Vorbehandlung und vorhergehende Behandlung mit Wasser, einer Säure oder Base oder ohne Zusatz weiterer Stoffe bei einer Temperatur von 840 bis 1150°C, gegebenenfalls unter einem Gasstrom aus Stickstoff oder Luft, kontinuierlich calciniert.

Das erfindungsgemäße Verfahren kann wie folgt durchgeführt werden:

Zur Veredelung im Sinne der vorliegender Erfindung ist jacken beliebige Eisenoxid geeignet, besonders geeignet sind durch aufarbeitung salzsaurer Beizlösungen hergestellte Eisenoxide, wie sie beispielsweise in der Stahlindustrie anfallen, insbesondere durch Sprühröstung salzsaurer Beizlösungen (Ruthner-Verfahren) hergestellte Eisenoxide.

Das Eisenoxide wird ohne Vorbehanlung, d.h. beispielsweise, ohne mechanische Vorbehandlung, bevorzugt trocken, d.h. ohne vorhergehende Behandlung mit Wasser, einer Säure oder-Base oder irgend einem anderen Stoff einer kontinuierlichen Calcinierung bei einer Temperatur von 700 bis 1200°C, bevorzugt 840 bis 1150°C, besonders bevorzugt 850 bis 1100°C, insbesondere 860 bis 1000°C in der Regel für 0,1 bis 24 h, bevorzugt 0,25 bis 10 h, besondere bevorzugt 0,3 bis 24 h, besondere 0,5 bis 1,5 h, unterzogen. Zur Calcinierung eignen sich alle bekannten Öfen. Die Calcinierung erfolgt kontinuierlich, beispielsweise in Drehrohröfen oder in Bandcalcinierern. Die Calcinierung kann bei nur einer Temperatur oder in Stufen über verschiedene Temperaturen oder über eine kontinuierliche Temperaturrampe durchgeführt werden. Bei der Calcinierung in Drehrohren sollten Klopfvorrichtungen verwendet werden, die ein Klebe des Eisenoxids an der Drehrohrwand vermeiden und für eine kontinuierliche Förderung des Eisenoxids sorgen. Vorteilhaft wird die Calcinierung in glatten Drehrohren ohne Einbauten durchgeführt, wobei die verweilzeit durch die Drehgeschwindigkeit, zulaufgesehwindigkeit und Neigung des Drehrohrs eingestellt werden kann. Vorteilhaft wird die Calcinierung weiterhin unter einem Gastrom, beispielsweise Stickstoff oder Luft durchgeführt, um freiwerdende Chlorverbindungen auszutreiben und zweckmäßigerweise in einer nachgesehalteten Abgaswäsche zu entfernen. Der Chloridgehalt lässt sich vorteilhaft in in sich ruhender Schüttung, also beispielsweise bei Calcinierung auf einem Bandcalcinierer reduzieren. Bei Calcinierung in bewegter Schüttung, beispielsweise in einem Drehrohr können zur Verringerung des Chloridgehalts vergleichsweise etwas höhere Temperaturen als in ruhender Schüttung erforderlich sein, was zu einer vergleichsweise weiter verringerten BET-Oberfläche führen kann. In Abhängigkeit von dem bevorzugten Verhältnis von Chloridgehalt und BET-Oberfläche kann es deshalb vorteilhaft sein, wahlweise in ruhender oder bewegter schüttung zu arbeiten.

Es können jedoch geringe Mengen von Wasser, Säuren, Basen oder organischen Verbindungen, soweit sie die Eigenschaften des veredelten Eisenoxids gegenüber einer trocknen Veredlung nicht negativ beeinflussen, zugesetzt werden. Vorzugsweise wird handelsübliches Eisenoxid ohne jegliche Vorbehandlung calciniert.

Als Eisenoxide für die erfindungsgemäße Veredelung eignen sich alle Eisenoxide, unabhängig von deren Herstellung. Geeignet sind beispielsweise natürliche, bevorzugt technisch hergestellte sowie handelsübliche Eisenoxide, insbesondere durch Aufarbeitung falzsaurer Beizlösungen hergestellte Eisenoxide. Diese können als Verunreinigung beispielsweise einen Restehloridgehalt und/oder Verbindungen von Titan, Mangan, Aluminium, Chrom, Phosphor, Zink, Kupfer, Molybdän, Wolfram, Silicium, Nickel, Magnesium, Kalium, Natrium, Cobalt, Vanadin, Zirkon, Niob, Schwefel, Lanthan, Blei, Zinn und/oder Calcium enthalten. Insbesondere eignen sich Eisenoxide, die durch Sprühröstung von salzsauren Beizlösungen in der Stahlindustrie hergestellt werden und als Fe₂O₃ mit einem Restchloridgehalt zwischen 0 und 10000 ppm, bevorzugt zwischen 50 und 5000 ppm und besonders bevorzugt zwischen 500 und 2000 ppm, üblicherweise in Hämatit-Kristallstruktur und einer BET-Oberfläche von typischerweise 3 bis 5 m²/g vorliegen.

Die erfindungsgemäß veredelten Eisenoxide haben in der Regel einen Restchloridgehalt von weniger als 400 ppm, bevorzugt wenger als 300 ppm und besonders bevorzugt weniger als 250 ppm, insbesondere weniger als 200 ppm. Die mittlere Partikelgröße, bestimmt durch Laserbeugung wie untenstehend beschrieben, beträgt in der Regel mehr als 5 µm, also 5,1 bis 200 µm, bevorzugt 8 bis 100 µm, besonders bevorzugt 10 bis 80 µm und ganz besonders bevorzugt 12 bis 30 µm, und der Feinanteil mit Partikelgrößen von weniger als 1 µm in der Regel weniger als 15 Gew.-%, bevorzugt weniger als 10 Gew.-%, besonders bevorzugt weniger als 5 Gew.-%. Die BET-Oberfläche der erfindungsgemäß behandelten Eisenoxide liegt in der Regel bei 0,4 bis 5 m²/g, bevorzugt 0,4 bis 3,5 m²/g, besonders bevorzugt 0,5 bis 3 m²/g, und insbesondere 0,6 bis 2,5 m²/g, ganz besonders bevorzugt 0,7 bis 2 m²/g. Die erfindungsgemäß behandelten Eisenoxide haben in der Regel eine Hämatitstruktur. Sie können für eine Reihe technischer Anwendungen beispielsweise Pharmazeutika, Kosmetika, Magnetbandbeschichtungen, chemische Reaktionen, Katalysatoren oder zur Herstellung von Katalysatoren, insbesondere für die Herstellung von Katalysatoren zur Dehydrierung von Ethylbenzol zu Styrol, verwendet werden.

### Beispiele

Im Beispiel 1 und dem Vergleichsbeispiel A wurde das Eisenoxid Type HP (Hösch Premium) der Firma Thyssen-Krupp als Einsatzstoff verwendet, das nach dem sogenannten Ruthner-Verfahren durch Sprühröstung salzsaurer Eisenlösungen hergestellt wird. Die Bestimmung des Restchloridgehalts erfolgte in allen Fällen coulometrisch. Die Partikelgröße des Eisenoxids wurde mit dem Gerät Mastersizer S der Fa. Malvern (Linse: 300 RFmm, Messbereich 0,05 bis 880 mm) durchgeführt. Dazu wurde das Modul MS17 eingesetzt. Hierbei handelt es sich um einen ProbevorlagenbehälEer zur Dispergierung in wässrigem Medium mit eingebautem Flügelrührer, integriertem Ultraschallgeber und Umlaufpumpe. Vor der Messung wurde das integrierte Ultraschallbad (Einstellung 100 %) in Betrieb genommen und nach 5 min. Dispergierzeit die Messung bei laufendem Ultraschall vorgenommen. Spezifische BET-Oberflächen wurden entsprechend der Vorschrift DIN 66133, Porenvolumina und mittlere Porenradien wurden entsprechend der Vorschrift DIN 66131 bestimmt.

### Beispiel 1

Das Eisenoxid wurde in einem Drehrohr kontinuierlich unter einem Luftstrom calciniert. Das Drehrohr war mit drei Klopfvorrichtungen ausgestattet. Die Wandtemperatur des Drehrohrs betrug 970°C, die Verweilzeit des Eisenoxids betrug ungefähr eine Stunde.

Die physikalischen Eigenschaften des erfindungsgemäß vorbehandelten Eisenoxids des Beispiels 1 sind in Tabelle 1 zusammengefasst und denen des nicht veredelten Eisenoxids gegenübergestellt.

**Tab. 1 Eigenschaften eines nach dem Ruthner-Verfahren hergestellten und kommerziell angebotenen Eisenoxids (Type HP der Fa. Thyssen-Krupp) und daraus durch die erfindungsgemäß Veredelung hergestellten Eisenoxids des Beispiels 1.**

| Eisenoxid | Rest-Chlorid [ppm] | mittlere Partikelgröße [µm] | Feinanteil kleiner 1 µm [Gew.-%] | BET-Oberfläche [m²/g] |
|---|---|---|---|---|
| Unbehandelt type HP (Thyssen-Krupp) | 1400 | 11 | 15 | 4,3 |
| Beispiel 1 | 190 | 13 | 1,9 | 0,9 |

## Patentansprüche

1. Verfahren zur Veredelung von Eisenoxiden, **dadurch gekennzeichnet, dass** man Eisenoxide aus der Sprühröstung von salzsauren Beizlösungen einsetzt und diese ohne mechanische Vorbehandlung und vorhergehende Behandlung mit Wasser, einer Säure oder Base oder ohne den Zusatz weiterer Stoffe bei einer Temperatur von 840 bis 1150 °C, gegebenenfalls unter einem Gasstrom aus Stickstoff oder Luft, kontinuierlich calciniert.

2. Verfahren nach Anspruch 1, **dadurch gekennzeichnet, dass** man Eisenoxide bei einer Temperatur von 850 bis 1000 °C calciniert.

3. Verfahren nach Anspruch 1 oder 2, **dadurch gekennzeichnet, dass** die Calcinierung in Drehrohröfen erfolgt und dass Drehrohre mit Klopfvorrichtungen verwendet werden, die ein Kleben des Eisenoxids an der Drehrohrwand vermeiden.

## Claims

1. A process for upgrading iron oxides, **characterized in that** iron oxides from the spray roasting of hydrochloric acid pickling wastes are used and are continuously calcined at 840 to 1150°C, optionally under a gas stream composed of nitrogen or air, without mechanical pretreatment and preceding treatment with water, an acid or base or without the addition of further substances.

2. The process according to claim 1 which is **characterized in that** iron oxides are calcined at 850 to 1000°C.

3. The process according to claim 1 or 2 which is **characterized in that** the calcination is carried out in rotary tube ovens and **in that** the rotary tubes used are equipped with tappers which prevent sticking of the iron oxide to the wall of the rotary tube.

## Revendications

1. Procédé de raffinage d'oxydes de fer, **caractérisé en ce que** des oxydes de fer issus du grillage par pulvérisation de solutions de décapage chlorhydriques sont utilisés et ceux-ci sont calcinés en continu à une température de 840 à 1 150°C, éventuellement sous un courant gazeux d'azote ou d'air, sans prétraitement mécanique et traitement préalable avec de l'eau, un acide ou une base, ou sans ajout de substances supplémentaires.

2. Procédé selon la revendication 1, **caractérisé en ce que** des oxydes de fer sont calcinés à une température de 850 à 1 000 °C.

3. Procédé selon la revendication 1 ou 2, **caractérisé en ce que** la calcination a lieu dans des fours tubulaires rotatifs et **en ce que** des tubes rotatifs munis de dispositifs secoueurs sont utilisés, qui évitent une adhésion de l'oxyde de fer sur la paroi du tube rotatif.
